# EUROPEAN PATENT APPLICATION

(11) **EP 2 352 028 A1**
(43) Date of publication of application: **03.08.2011**
(21) Application number: 09829020.8
(22) Date of filing: 19.11.2009
(51) Int. Cl.: G01N 33/543

(54) **IMMUNOCHROMATOGRAPHIC MEDIUM AND IMMUNOCHROMATOGRAPHIC METHOD**

(30) Priority: 28.11.2008 JP 2008303998
(71) Applicant: Konica Minolta Medical & Graphic, Inc., Tokyo 191-8511 (JP)
(72) Inventor: TSUKADA Kazuya, Hino-shi Tokyo 191-8511 (JP); FUJII Tuneko, Hino-shi Tokyo 191-8511 (JP)
(74) Representative: Gille Hrabal Struck Neidlein Prop Roos
(86) International application number: PCT/JP2009/069609
(87) International publication number: WO 2010/061772

(57) **Abstract**

Disclosed are an immunochromatographic medium and an immunochromatographic method both of which, even when colors that can be detected by colored latex particles, i.e., more than four items, are to be detected, can detect and quantify the multiple items simultaneously. The immunochromatographic medium is **characterized by** comprising a support, a label-attached reagent which is immobilized on a labeling reagent and is arranged on the support, and a detection part on which a substance capable of binding to a substance to be detected in a sample is immobilized, wherein the labeling reagent is a fluorescent nano-particle and the detection part has a dot-like pattern,

## Description

### FIELD OF THE INVENTION

The present invention relates generally to an immunochromatographic method employing an immunochromatographic medium for detecting and discriminating, on one support, plural items of plural substances to be analyzed (refereed to as also analytes), if present, in a single fluid sample.

### TECHNICAL BACKGROUND

The field of rapid diagnostic testing has evolved over many years to permit the detection of analytes in various types of samples. Many of the rapid immunoassay based tests include a solid housing encasing a test strip. However, recently, an immunochromatographic assay have been developed which does not have a solid housing. Such tests, referred to as dipsticks or urinary test strips, can be dipped directly into a tube containing a pre-determined amount of the target liquid sample. The end of the dipstick with a sample-receiving pad is generally brought into contact with a liquid sample, and the liquid migrates up the flow path. One disadvantage of the current immunochromatographic dipsticks is that they can only detect the presence of one analyte. For example, because only one analyte at a time can be evaluated, it is recognized that the use of rapid test of a single analyte is often limited. Therefore, the increase in a necessary amount of a sample increases the load on sampling, or the dilution of a sample makes it difficult to detect a minute amount of analytes. When the addition of a sample to the center is not well controlled, there is possibility that the same amount of the sample cannot be developed on each strip, resulting in lowering of ease of handling. Further, difference between lots of the strips is increased, resulting in the possibility that reliability of data obtained lowers.

In contrast, an assay for detecting plural items has been proposed (see, for example, Patent Document 1). However, there is limitation in the assay to the number of lines on one strip. In the strip disclosed in that patent document, in which a control zone and a test zone are alternately arranged, it is considered that the substances to be detected are limited to three kinds thereof. Further, it is considered that there occurs a great difference in the concentration of a sample to react between at the line arranged at a position closer to the sample addition portion and at the line arranged at a position further from the sample addition portion, and therefore, there is possibility that sensitivity differs due to the position where the lines are arranged.

### PRIOR ART

### PATENT DOCUMENTS

Patent Document 1: Japanese Unexamined Patent Application Publication (Translation of PCT Application) No. 2007-506115

### DISCLOSURE OF THE INVENTION

### PROBLEMS TO BE SOLVED BY THE INVENTION

An object of the invention is to provide an immunochromatographic medium and an immunochromatographic method in which, even when plural items exceeding four items are detected which are the number of colors that can be detected by colored latex particles, the plural items can be detected and quantified simultaneously.

### MEANS FOR SOLVINGTHE ABOVE PROBLEMS

The above object of the invention can be attained by the following constitutions.
1. An immunochromatographic Medium comprising a support, a label-attached reagent immobilized on a labeling agent, and detection portions on which a material capable of combining with a substance to be detected in a sample is immobilized, the label-attached reagent and the detection portions being provided on the support, featured in that the labeling agent is a phosphor nanoparticle and the detection portions are shaped in the form of dots.
2. The immunochromatographic medium of item 1 above, featured in that the number of the dots of the detection portions is from 3 to 30.
3. An immunochromatographic method employing the immunochromatographic medium of item 1 or 2 above, the method featured in that it comprises reacting a label-attached reagent, which is immobilized on a labeling agent and which is capable of combining with a substance to be detected in a sample, with a substance to be detected, thereby obtaining a chromatographic solution; causing the chromatographic solution to migrate on the immunochromatographic medium to detection portions on which a material capable of combining with the substance to be detected is immobilized, and capturing and detecting the substance to be detected at the detection portions.
4. The immunochromatographic method of item 3 above, featured in that the labeling agent is a quantum dot.
5. The immunochromatographic method of item 3 or 4 above, featured in that three to fifteen items of the substance to be detected can be detected simultaneously.

### EFFECTS OF THE INVENTION

The present invention can provide an immunochromatographic medium and an immunochromatographic method each of which can detect and quantify plural items simultaneously.

### BRIEF EXPLANATION OF THE DRAWINGS

Fig. 1 shows a plan view and sectional view of one embodiment of an immunochromatographic assay medium of the invention.
Fig. 2 shows a detection method employing one embodiment of an immunochromatographic assay medium of the invention.
Fig. 3 shows a schematic view of one embodiment of an immunochromatographic medium employing a membrane.

### PREFERRED EMBODIMENT OF THE INVENTION

The immunochromatographic medium and immunochromatographic method of the invention employ one strip of the immunochromatographic medium which uses a phosphor nanoparticle, whereby simultaneous analysis of plural items can be carried out easily and quantitatively. That is, the use of the immunochromatographic medium of the invention enables multicolor light emission on excitation with light with one wavelength, and plural items can be simultaneously detected without increasing the number of operation procedures. Further, when the detection portions are shaped in the form of lines, the upper limit of the number of the detection portions is three or four, however, the detection portions shaped in the form of dots enable provision of more detection portions, since the area occupied by the dots is small. Accordingly, in the immunochromatographic medium of the invention, three, four or more kinds of items can be detected employing one strip, and therefore, can be detected without varying the amount of a sample to be added. Further, dilution of a sample is unnecessary, and therefore, plural items can be detected with high sensitivity.

The support used in the immunochromatographic medium of the invention is required to stably and rapidly diffusion-transfer a composite of a substance to be detected and a detecting reagent in the presence of a solvent to certainly reach a chromatographic detection portion and therefore, it is necessary that the support have a pore diameter larger than that of the composite. Typical examples thereof include carriers such as a glass fiber sheet, a porous material such as paper or nitrocellulose, silica used in a fiber matrix or a thin layer chromatography, minute particulate cellulose, and nylon-6,6. Preferred porous materials, which are used to cany out a lateral flow support assay with high sensitivity and high specificity, are preferably celluloses with minute pores, for example, a cellulose ester of cellulose with an aliphatic carboxylic acid such as alkane carboxylic acid, and more preferably materials with minute pores made of nitrocellulose. A mixture of the cellulose ester described above and nitrocellulose also is preferably used. The shape of the immunochromatographic medium having the support as described above is ordinarily belt-shaped, and the chromatographic detection portions on the medium are shaped in the form of dots. The length and width, by which a chromatographic reaction solution transfers on the medium of the invention, are ordinarily from 1 to about 10 cm, and ordinarily from 0.5 to 2 cm, respectively.

In the invention, the detection portions shaped in the form of dots allow for simultaneous detection ofplural items. If the detection accuracy can be secured, it is possible to provide many dots on one immunochromatographic medium. The quantum dots for the detection have a dot number of preferably from 3 to 30, and more preferably from 5 to 30, in view of the effectiveness, ease of visibility or ease of handling.

The shape of the dots is preferably in the form of a rectangle or a circle, and the dot size is not specifically limited but is preferably from 1 to 5 mm in view of visibility, ease of measurement or case of handling. Further, any two dots adjacent to each other are provided at an interval in which immobilized antibodies do not overlap with each other. The interval can be from 1 to 2 mm.

It is preferred that the dots are two-dimensionally provided substantially at regular intervals in view of ease of measurement or certainty of visibility. Concretely, the dots can be provided in the form of matrix, and the row number and the column number of the dot matrix can be from 1 to about 6, respectively.

The dots are provided in a material covering an absorption pad, and an antibody for immobilization can be immobilized thereon by jetting. Thereafter, the provided dots can be protected employing a laminate and the like.

As samples in the invention, various solutions can be employed, and liquid samples derived from a living thing such as blood, blood plasma, blood serum, saliva, urine, milk, pleural fluid, mucous membrane, celebrospinal fluid, peritoneal fluid, pus, or excrements can be used. Further, the samples may be those derived from fermentation process, cell culture, chemical reaction mixtures, and the like. The samples may be liquid ones other than the biological fluids or physiological fluids, for example, samples for water or food product examination or samples for water or soil examination. A subject for detection may be one, in which a material specifically combining with the subject (a material capable of specifically combining with a substance to be detected) is contained, for example, polypeptides, proteins, composite proteins, polysaccharides, lipids, composite lipids, hormones, and nucleic acids. As a material specifically combining with these, there are mentioned an antibody, a receptor or an aptamer.

### [Labeling Agent]

The labeling agent implies a material for labeling which is capable of being attached to a specific binding constituent (antibody) and detected. In the invention, phosphor nanoparticles are used as such a material. The phosphor nanoparticles have an advantage that in spite of their small size, high luminescence intensity can be obtained. Quantum dots are preferably employed. The quantum dots provide high luminescence intensity due to the quantum size confinement effect, and at the same time quantum dots having the same composition but a different size have an advantage of emitting light with a different wavelength, and further have a great advantage of emitting multicolor lights in the visible to infrared wavelength regions due to the narrow half-value width of the wavelength of the emission light. The above advantages of the dots in the invention exert the effects of the invention more efficiently.

As the phosphor nanoparticles, there can be employed quantum dots exhibiting the quantum size effect, activator type phosphor nanoparticles in which incorporation of an activator causes fluorescence, and host excitation type nanoparticles.

In the invention, it is especially preferred that the semiconductor nanoparticles as described later are employed as the quantum dots.

As materials for the semiconductor nanoparticles in the invention, well known various fluorescence emission compounds and their materials. For example, the semiconductor nanoparticles can be prepared employing various semiconductor materials, which are well known as materials for conventional semiconductor nanoparticles. Examples thereof include semiconductor compounds comprising Group IV elements, elements of Groups II and VI and elements of Groups III and V of the periodic table; and material compounds containing the element constituting the semiconductor compounds.

Examples of the semiconductor materials comprising elements of Groups II and VI include MgS, MgSe, MgSe, MgTe, CaS, CaSe, CaTe, SrS, SrSe, SrTe, BaS, BaSe, BaTe, ZnS, ZnSe, ZnTe, CdS, CdSe, HgS, HgSe, and HgTe.

Examples of the semiconductor materials comprising elements of Groups III and V include GaAs, GaN, GaPGaSb, InGaAs, InP, InN, InSb, InAs, AlAs, Alp, AlSb, and AlS.

Of the semiconductor materials comprising Group IV, Ge and Si are especially preferred.

Examples of the quantum dots described above include CdSe, CdTe, InP, InN, InAs, Cds, Si, and Ge. Si and InP are especially preferred in view of the handling property and environmental acceptability, and sufficiently provide the effects of the invention (simplicity and convenience).

The quantum dots in the invention sufficiently provide the quantum confinement effect, and are preferably core-shell type quantum dots in view of high luminance. For example, when the core is formed of silicon, silica is employed as the shell, and when the core is formed of CdSe, ZnS is employed as the shell. In order to prepare the core of quantum dots, conventional various preparation methods can be employed and the optimum preparation method can be employed according to the selected composition of the dots. Regarding preparation of the shell, the optimum preparation method can be employed according to the selected composition of the core. In the invention, core-shell type silicon quantum dots are preferably employed. In the preparation of the core of the silicon quantum dots are preferably employed a high frequency sputtering method in which the particle size of the quantum dots can be easily controlled and a preparation method comprising the acid treatment and stirring treatment changing the shape of the silicon quantum dots from the film shape to the particle shape. The latter method has characteristics in that the shape of silicon quantum dots are changed from the film shape to particle shape, and dispersed in a solution to cause fluorescence. In formation of the shell, when silicon quantum dots are employed, natal oxidation or annealing oxidation may be carried out at optimum temperature and at optimum oxygen concentration to form an oxidation film (silica film).

The preferred core-shell type silicon quantum dots in the invention are featured in core-shell type silicon quantum dots composed of a core containing silicon and a shell containing silicon oxide. In order to enhance the luminescence intensify, it is preferred that an atom such as Be, Ga, Mg or Mg is incorporated in a small amount into at least one of the core and the shell.

The quantum dots in the invention are preferably those which have an average particle size of from 2 to 10 nm and emit visible to infrared light with a wavelength of 400 to 900 nm when excited by ultraviolet to visible light with a wavelength of from 200 to 500 nm.

In the invention, the average particle size of the quantum dots should be determined in terms of three dimensions, but the determination is difficult since the dots are too small, and actually, the average particle size is determined employing a two-dimensional particle image. It is preferred that particles at various portions are photographed employing a transmission electron microscope (TEM) to obtain many electron micrographs of the particles, and the average particle size is determined from them.

Adjustment of the particle size can be carried out according to variation of sputtering conditions. The particle size can be controlled, varying high frequency power or atmospheric gas pressure (pressure applied during preparation, argon gas pressure in the preparation in the invention) as the sputtering conditions, as well as the area ratio of the silicon chip and silica target material. Herein, the high frequency power is varied in the range of from 10 to 500 W, and the gas pressure in the range of from 1.3 x 10⁻² to 1.3 x 10 Pa (from 1 x 10⁻⁴ x to 1 x 10⁻¹ torr).

As long as an organic molecule is one which is capable of combining a labeling substance (a phosphor particle, etc.) of a labeling agent with a reagent (a molecule labeling substance), the organic molecule is not specifically limited. For example, albumin, myoglobin, casein and the like among proteins are suitably employed. Further, avidin, which is one kind of protein, is preferably used in combination with biotin. The combining manners in the combination above include covalent bonding, ionic bonding, hydrogen bonding, coordination bonding, physical adsorption or chemical adsorption, although not specifically limited. From the viewpoint of bonding stability, bonding featuring a strong bonding force such as covalent bonding is preferred.

As the organic molecule, one having a mercapto group (a thiol group), a carboxyl group or an amino group is preferably employed. Typical examples of the organic molecule include mercaptopropionic acid, mercaptoundecanoic acid, aminopropane thiol, mercaptopropyltriethoxysilane and aminopropyltriethoxysilane.

### [Label-Attached Reagent]

The label-attached reagent in the invention is one in which an organic molecule modified labeling agent is combined with a reagent (also referred to as a molecule labeling substance) through the organic molecule. The label-attached reagent in the invention specifically combines and/or reacts with a living substance as a target through the reagent (preferred also as the molecule labeling substance), whereby the living substance can be labeled. The reagent in the label-attached reagent in the invention implies a material capable of combining with a substance to be detected, for example, the antibody described previously (referred also as a specific binding constituent), a receptor or an aptamer. In the invention, a material labeled with the labeling agent is in advance immobilized as the label-attached reagent in the invention on a portion where the material is reacted with a sample containing a substance to be detected.

Examples of the reagent include a nucleotide chain, an antibody, an antigen and cyclodextrin.

Concretely, in the case where core-shell type silicon quantum dots are processed with mercaptoundecanoic acid, avidin and biotin can be used as the organic molecules. In this case, the carboxyl group of the nanoparticles suitably combines covalently with avidin, the avidin selectively combines with biotin, and the biotin further combines with the reagent (referred also as the molecule labeling substance) to obtain a label-attached reagent.

### <Principle of Membrane Assay Method>

Next, the determination principle in the invention will be explained briefly.

The "immunochromatographic method" of the invention is a method which detects a substance to be detected contained in a sample in a short period of time, employing a membrane on which a specific binding constituent specifically combining with the substance to be detected is solidified. In more detail, the method is a so-called a sandwich assay method in which, employing a label reagent specifically combining with a substance to be detected in a sample and a membrane, comprising a detection portion on which a specific binding constituent capable of specifically combining with the substance to be detected in a sample is immobilized, a composite, which is composed of the specific binding constituent, the substance to be detected and the label reagent, is formed on the detection portion of the membrane, whereby the substances to be detected is detected by quantum dots combining with the label reagent.

As a reaction of the substance to be detected with the specific binding constituent or the label reagent, there are an antigen-antibody reaction, a reaction of a receptor with another receptor, a specific binding reaction of biotin with avidin and a reaction between DNAs having a complementary sequence. The antigen-antibody reaction is preferred.

As the membrane assay method, two kinds of membrane immunochromatographic assay methods, i.e., a flow through type membrane assay method and a lateral flow type membrane assay method are preferably employed.

The flow through type membrane assay method is one in which a solution containing a substance to be detected is caused to migrate in the perpendicular direction on a membrane on which a specific binding constituent combining with the substance to be detected or a material for detection is coated, a composite is formed on the membrane, which is composed of the specific binding constituent combining with the substance to be detected, the substance to be detected and a second labeling agent specifically combining with the substance to be detected, and the label is detected and quantified, whereby the substance to be detected is detected or quantified.

The lateral flow type membrane assay method is carried out employing the same membrane as described above. This method is different from the flow through type membrane assay method in that a solution containing a substance to be detected is caused to migrate in the horizontal direction on the same membrane as described above, and the detection principle of the substance to be detected is the same as the flow through type membrane assay method. Of the above two assay methods, the lateral flow type membrane assay method is preferred in view of simplicity and short time of detection.

### <Specific Binding Constituent Specifically Combining With Substance To Be Detected>

A specific binding constituent for capturing a substance to be detected is a material, which combines with a substance to be detected through a specific reaction such as an antigen antibody reaction to form a composite. Therefore, as a matter of course, a specific binding constituent, which is different due to kind of a substance to be detected, is employed. Generally, when substances to be detected are bacilli, virus, hormone or other clinical markers, a polyclonal antibody or a monoclonal antibody is employed which specifically reacts with and combines with these substances. Further, when substances to be detected are antibodies, a virus antigen, virus hollow particles, a recombinant coli expression protein and a recombinant yeast expression protein, are employed which specifically react with and combine with these substances.

A method which immobilizes such a specific binding constituent onto the support described previously may be one which is carried out through physical absorption or chemical bonding. Preparation of the support to which the specific binding constituent is bound is carried out, for example, by a method which adsorbs onto a support a solution in which the specific binding constituent is diluted in a buffering solution, followed by drying.

In the invention, in order to detect or quantify plural kinds of the substances to be detected, a specific binding constituent specifically combining with each of the substances is required to be immobilized on a support, and each of the specific binding constituents is required to be immobilized on a different section on the support.

### <Substance To Be Detected>

As the substances to be detected in the invention there are mentioned pathogenic microorganisms such as influenza virus antigen, antibodies to the pathogenic microorganisms, peptide hormones, steroids, biologically active amines, vitamins, prostaglandins, antibiotic substances such as tetracyclines, toxins produced by bacteria, various tumor markers, agricultural chemicals, and nucleotides complementary to nucleic acids derived from pathogenic microorganisms. The assay method in the invention is especially effective in detecting a pathogenic organism such as influenza virus or RS (Respiratory Syncitial) virus, which widely spreads and is required to be identified rapidly.

The assay method of the invention is especially effective for a substance to be detected which is likely to produce a false positive result.

When two or more kinds of substances to be detected are at least two kinds of the substances selected from type A influenza virus, type B influenza virus and RS virus, a false positive result can be effectively prevented.

Analytes to be analyzed by means of the assay method of the invention include biological samples such as pharyngeal swab, nasal swab, nasal suction fluid, excrement suspension, blood plasma, blood serum, urine, saliva, amniotic fluid, celebrospinal fluid, pus, organ extract solutions, and extract solutions of various tissues; food extract solutions; culture supernatants; tap water; sewerage; lake water, river water; seawater; soil extract solutions and mud extract solutions, but are not limited thereto. Particularly, pharyngeal or nasal swab, nasal suction fluid, nasal rinse and excrement suspension contain high viscosity components derived from the biological samples as described above at a high content and are likely to cause the false positive, and therefore, the assay method of the invention is effective in evaluation of these analytes. Further, the assay method is more effective in evaluation of pharyngeal or nasal swab, nasal suction fluid and nasal rinse among these analytes.

### EXAMPLES

Next, the present invention will be explained with reference to Examples, but the invention is not specifically limited thereto.

### <<Determination of Humane Cytokine>>

### [Example 1]

### (1) Preparation of Monoclonal Antibody

A spleen was extirpated from a BALB/c mouse in which purified IL-1a, IL-1b, IL-2, IL-4, IL-6, IL-7, IL-8, IL-10, IL-12q70, GM-CSF, IFNγ, and TNFα antigens were immunized and maintained for a prescribed period, and fused with a mouse myeloma cell (p3 x 63) according to a Kohler et al. method (Kohler et al., Nature, vol. 256, p495-497 (1975). The resulting fused cell (hybridoma) was maintained at 37 °C in an incubator, whereby purification (cloning) of the cell was carried out. Two lines of each of the resulting cells were injected in the peritoneal cavity of a BALB/c mouse subjected to pristane treatment. About two week after, the antibody-containing ascites fluid was taken, and purified according to affinity purification employing Protein A column chromatography produced by GE) to purify IgG. Thus, two of the purified antibodies to each antigen were prepared.

### (2) Preparation of Quantum Dots (Core-Shell Type Silicon Quantum Dots)

The core-shell type silicon quantum dots in the invention was core-shell type silicon quantum dots prepared according to a high frequency sputtering method, and prepared through the following preparation procedures.

### Procedure (1)

Employing silicon and silica as a target material, amorphous silicon oxide film was formed on a substrate according to a high frequency sputtering method. The high frequency sputtering apparatus was composed of a vacuum chamber on the side lower part with an introduction inlet and an exhaustion outlet of argon gas; a substrate holder fitted on the upper part of the vacuum chamber through an insulation material, which is cooled with chilled water introduced and discharged from a cooling tube; and a high frequency electrode with a cathode shield fitted on the lower part of the vacuum chamber through an insulation material, which is cooled with chilled water introduced and discharged from a cooling tube.

In the above apparatus, an argon gas was introduced into the vacuum chamber from the argon gas introduction inlet, ionized through a high frequency controller to produce an ionized argon gas. The resulting ionized argon gas was caused to collide with a silicon chip and quartz glass as the target materials on the high frequency electrode to release atoms or molecules from the target materials. The released atoms or molecules were deposited on the substrate held on the substrate holder to form an amorphous silicon oxide film.

The particle size was controlled varying an area ratio of the silicon chip and the quartz glass constituting the target materials, and the area ratio of each particle was varied so that the quantum dot size as shown in Table 1 was obtained. The area ratio was ordinarily from 1 to 50%, and preferably from 5 to 30% in view of production efficiency or the particle size distribution.

### Procedure (2)

The amorphous silicon oxide film was heat treated under an inert gas (argon gas) atmosphere to form silicon quantum dots (core part) with a pre-determined particle size in the amorphous silicon oxide film. During the heat treatment, the heat treatment temperature was in the range of from 1000 to 1100 °C, and adjusted due to an intended particle size. The heat treatment time was in the range of from 50 to 70 minutes, and adjusted due to an intended particle size.

### Procedure (3)

The amorphous silicon oxide film after heat treatment was subjected to hydrofluoric acid treatment to expose the silicon quantum dots.

Concretely, the substrate with a silicon oxide film containing the silicon quantum dots was adhered to an acrylic plate and placed in a vessel containing an aqueous hydrofluoric acid solution with the silicon oxide film facing downward. The concentration of the hydrofluoric acid was in the range of from 20 to 30%, and adjusted due to state of the silicon oxide film obtained.

The vessel containing an aqueous hydrofluoric acid solution was provided in a constant temperature water bath containing pure water equipped with a heater to exert hydrofluoric acid treatment. The above hydrofluoric acid treatment was carried out at 40 °C, and at a treating period of from 8 to 15 minutes.

In the above hydrofluoric acid treatment, hydrofluoric acid vaporized from the acid aqueous solution was adhered to the surface of the film and the surface of the silicon oxide part in the silicon oxide film was gradually etched. As a result, a large number of silicon quantum dots were exposed on the substrate in the aggregation state.

### Procedure (4)

The substrate with the exposed silicon quantum dots was immersed in a solvent to separate the silicon quantum dots from the substrate, thereby obtaining a silicon quantum dot dispersion solution. Concretely, the substrate with the aggregated and exposed silicon quantum dots was placed in a vessel containing ethanol. A magnetic stir bar was placed in the vessel, the dispersion solution was stirred with a stirrer, or alternatively, the vessel is placed on an ultrasonic cleaner and subjected to ultrasonic cleaning. During the above stirring treatment, the silicon quantum dots exposed on the substrate in the aggregation state were separated from the substrate and dispersed in ethanol. The stirring duration of the stirring treatment was from 60 to 600 seconds.

### Procedure (5)

The silicon quantum dots were naturally oxidized under an oxygen atmosphere or thermally oxidized by heat application heat to form a shell composed of silicon oxide around the core composed of the silicon quantum dots, thereby obtaining core/shell type silicon quantum dots. Concretely, the silicon quantum dots dispersed in the ethanol were naturally oxidized under an oxygen atmosphere or thermally oxidized by heat application. During the oxidation treatment, a shell composed of silicon oxide was formed around the core composed of the silicon quantum dots.

In the above treatment, the treatment temperature was in the range of from 25 to 35 °C, and the optimal duration of the treatment was selected from the treatment duration of from 8 to 14 hours.

### Procedure (6)

The resulting core/shell type silicon quantum dots were reacted with a hydrogen peroxide solution to hydroxylate the crystal surface. The core/shell type silicon quantum dots subjected to hydroxylation can easily react with a silane coupling agent and the like.

Concretely, the core/shell type silicon quantum dots were reacted with a hydrogen peroxide solution to hydroxylate the crystal surface, where the treatment temperature was adjusted at a temperature of from 25 to 35 °C, and the optimal duration of the treatment was selected from the treatment duration of from 10 to 30 minutes. The hydrogen peroxide concentration of the hydrogen peroxide solution was selected from the range of from 20 to 30%.

### Procedure (7)

The resulting hydroxylated core/shell type silicon quantum dots were washed with hot water. Concretely, the washing time was from 3 0 to 240 seconds, and the washing temperature was from 75 to 80 °C. The resulting quantum dots were determined according to the following method. The average particle size of the quantum dots is determined as follows. The quantum dots are photographed employing a TEM to obtain the electron micrograph, and the section area of a sufficient amount of the quantum dots in the electron micrograph is measured. Then, the diameter of the circle having the same area as the measurement is determined and the arithmetic average of the diameters is designated as the average particle size. The number of cluster particles to be photographed by TEM is preferably not less than 100, and more preferably 1000. In the invention, the arithmetic average of the diameters of 1000 particles is defined as the average particle size. The particle size determined is shown in Table 1.

The core/shell type silicon quantum dots as the labeling agent in the invention was combined with the functional substance (molecule labeling substance) of a label-attached reagent through an organic molecule through the procedure described later to prepare a label-attached reagent (living substance labeling agent).

### Procedure (8)

The core/shell type silicon quantum dots, which were washed with hot water, were combined with an organic molecule through the hydroxyl group of the quantum dots.

Concretely, the quantum dots as shown in Table 1 and aminopropyltriethoxysilane were mixed and stirred in ethanol at room temperature to cause silane coupling reaction, whereby an amino group was introduced on the surface of the dots. The resulting reaction product and polyethylene glycol with a molecular weight of 1000, having N-hydroxysuccinimide group and maleimide group as a functional group on each end, were mixed and reacted with stirring at room temperature for 24 hours to obtain core/shell type silicon quantum dots combined with ethylene glycol as the organic molecule having a meleimide group as the end group (a labeling agent).

### (3) Preparation of Quantum Dot Labeling Antibody

The silicon quantum dots reacted with the organic molecule were combined with a molecule labeling substance such as an antibody or the like to obtain a label-attached reagent.

Concretely, as shown in Table 1, the core/shell type silicon quantum dots having a different particle size, which were combined with the polyethylene glycol having a meleimide group as the molecular end as an organic molecule, were reacted with different kinds of antibodies according to the following procedures. Thus, core/shell type silicon quantum dots with an antibody introduced in the end (label-attached reagent) were prepared.

### (Preparation of Quantum Dot Labeling Antibody Pad)

The quantum dot labeling antibody prepared above was jetted onto the entire surface of a cellulose unwoven fabric with a width of 15 mm, employing a positive pressure jetting device Biojet (Produced by Bio Dot Co., Ltd.), and dried spraying a 50 °C hot air for one minute. Thus, a quantum dot labeling antibody pad was prepared.

### (4) Preparation of Antibody to be immobilized on Membrane

Of the purified antibodies prepared in item (1) above, those which were not subjected to labeling processing were dialyzed in a fixative solution (10 mM Tris-HCl (pH 7.5)), subjected to 0.02 µm filtration processing, and diluted with the fixative solution. Thus, an antibody to be immobilized was prepared.

### (5) Preparation of Immunochromatographic Medium for Detection of Humane Cytokine

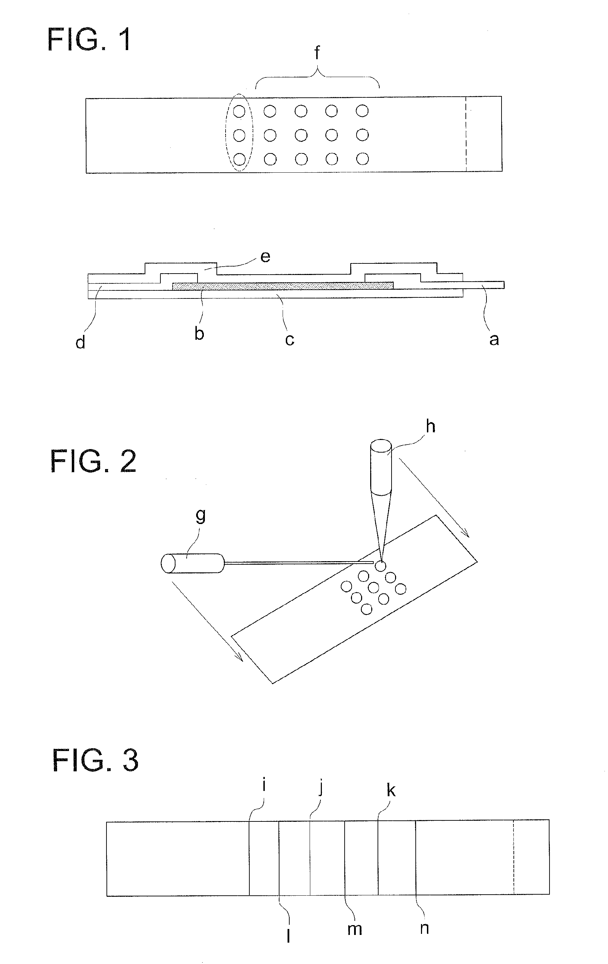

The immmunochromatographic assay medium for detection of humane cytokine having the same structure as shown in Fig. 1 was prepared and employed.

As the membrane, a nitrocellulose membrane sheet (white) with a size of 3 cm x 10 cm (produced by Millipore Co., Ltd.) was employed. As shown in Fig. 1, an anti-IL-1a antibody to be immobilized was coated in the form of dot at a position distant from one end (this end was designated as an upstream end, and the other end as a downstream end) on the long side of the sheet, employing a positive pressure jetting device Biojet (produced by Bio Dot Co., Ltd.). Then, an anti IL-1b antibody to be immobilized, an anti IL-2 antibody to be immobilized, an anti IL-4 antibody to be immobilized, and a control antibody exhibiting termination of reaction were coated in the perpendicular direction under that position in that order in the same way as the anti IL-1 a antibody. Further, an anti IL-6 to be immobilized was coated on the right side of the anti IL-1a antibody described above, and an anti IL-7 antibody to be immobilized, an anti IL-8 antibody to be immobilized, an anti IL-10 antibody to be immobilized, and a control antibody were coated in the perpendicular direction under the anti IL-6 antibody in that order. Further, an anti IL-12q70 to be immobilized was coated on the right side of the anti IL-6 antibody described above, and an anti GM-CS antibody to be immobilized, an anti IFNγ antibody to be immobilized, an anti TNFα antibody to be immobilized, and a control antibody were coated in the perpendicular direction under the anti IL-12q70 antibody in that order. After that, the coatings were dried by spraying a 45 °C hot air for ten minutes. Then, in order to fix the resulting material and enhance the strength, a plastic backing sheet (produced by Bio Dot Co., Ltd.) was laminated on the surface (designated as under surface) of the membrane opposite the surface (designated as upper surface) with the coatings.

Subsequently, the quantum dot labeling antibody pad prepared above was cut, and adhered on the upper surface of the membrane so that the pad overlapped with the membrane by 2 mm from the upstream end, thereby providing a sample dropping pad.

Subsequently, a cellulose filter paper with a size of 30 mm (width) x 10 cm (length) (produced by Watmann Co., Ltd.) was adhered on the upper surface of the membrane so that the filter paper overlapped with the membrane by 5 mm from the downstream end, thereby providing a sample absorption pad.

Subsequently, the entire upper surface was covered with a transparent plastic laminate (produced by Adhesive Research Co., Ltd.) except for an upstream end width of 7 mm of the sample dropping pad. Thus, an immunochromatographic medium as shown in Fig. 1 was prepared.

### (6) Preparation of Reaction Sample Solution for Chromatography

A humane cytokine in an amount of 500 pg/ml was mixed in a PBST buffer aqueous solution to prepare a reaction sample solution for chromatography.

### (7) Detection of Humane Cytokine

The reaction sample solution for chromatography prepared in item (6) above was dropped on the sample dropping portion of the immunochromatographic assay medium prepared in item (5) above and chromatographically developed. Ten minutes after, signals were detected at the detection portions. As is shown in Fig. 2, the detection was carried out employing a box type fluorescence detection apparatus equipped with an excitation light source capable of carrying out automatic scanning and a fluorescence detector capable of automatically scanning and detecting spots where luminescence light was emitted by the excitation light and of measuring the wavelength and the fluorescence intensity of the emitted luminescence, the apparatus being shielded from the outside light. The light wavelength of the excitation light source was set at 280 nm. Luminescence intensity was represented by a relative luminescence intensity when Alexa Flour 430 (produced by Invitrogen Co., Ltd.) was used as the quantum dots and the intensity of the luminescence emitted from the spot with the same area was set at 100. Regarding luminescence spot detectability due to automatic scanning, when a spot signal at the fluorescence detecting portion was clearly detected, it was evaluated as "A"; when the spot signal was not so clearly detected due to noise generation, it was evaluated as "B"; and when the spot signal was not detected, it was evaluated as "C". The results are shown in Table 1.

**Table 1**

| Sample No. | Label-attached Reagent (Antibody for Immobilization) | Average Particle Size (nm) of Core/Shell Quantum Dots | Luminescence Wavelength λmax (nm) | Luminescence Intensity*1 | Visual Observation/Detectability due to Automatic Detection*2 |
|---|---|---|---|---|---|
| 1 | Anti IL-1a Antibody | 2.2 | 350 | 500 | The spot can be clearly discrimmated/A |
| 2 | Anti IL-1b Antibody | 2.4 | 370 | 450 | The spot can be clearly discriminated/A |
| 3 | Anti IL-2 Antibody | 2.6 | 390 | 450 | The spot can be clearly discnmmated/A |
| 4 | Anti IL-4 Antibody | 2.8 | 410 | 420 | The spot can be clearly discriminated/A |
| 5 | Anti IL-6 Antibody | 3 | 430 | 400 | The spot can be clearly discriminated/A |
| 6 | Anti IL-7 Antibody | 3.2 | 450 | 400 | The spot can be clearly discriminated/A |
| 7 | Anti IL-8 Antibody | 3.4 | 470 | 380 | The spot can be clearly discriminated/A |
| 8 | Anti IL-10 Antibody | 3.6 | 490 | 350 | The spot can be clearly discriminated/A |
| 9 | Anti IL-12q70 Antibody | 3.8 | 520 | 350 | The spot can be clearly discriminated/A |
| 10 | Anti GM-CSF Antibody | 4 | 550 | 330 | The spot can be clearly discriminated/A |
| 11 | Anti IFNγ Antibody | 4.2 | 580 | 330 | The spot can be clearly disedminated/A |
| 12 | Anti TNFα Antibody | 4.4 | 610 | 330 | The spot can be clearly disedminated/A |

| | | | | | |
|---|---|---|---|---|---|
| *1: Luminescence intensity was represented by a relative luminescence intensity when Alexa Flour 430 (produced by Invitrogen Co., Ltd.) was used as the quantum dots and the intensity of the luminescence emitted from the spot with the same area was set at 100. *2: Regarding luminescence spot detectability due to the automatic scanning, when the spot signal at the fluorescence detecting portion was clearly detected, it was evaluated as "A"; when the spot signal was not so clearly detected due to noise generation, it was evaluated as "B"; and when the spot signal was not detected, it was evaluated as "C". | | | | | |

As is shown in Table 1, the cytokine detection method employing immunochromatograpbic medium of the invention can simultaneously detect twelve kinds of cytokines, each of which is detected without difficulty in discrimination as a clearly discriminated spot. The results show that the constitution of the present invention adds excellent function that can simultaneously detect many kinds of analytes in addition to in vitro diagnosis according to a conventional immunochromatographic method featured in simplicity, providing a high grade diagnosis method.

### [Comparative Example 1]

### (1) Preparation of Monoclonal Antibody

Those prepared in Example 1 were employed.

### (2) Preparation of Latex Particle Labeling Antibody

Each one of the purified antibodies prepared in item (1) above was dialyzed in a 50 mM MES (2-morpholinoethanesulfonic acid, monohydrate, produced by Dojin Kagaku Co., Ltd.) buffering solution (pH 6.0), mixed with colored polystyrene latex particles (Magshere, produced by Bangs Laboratories, Inc.) in the combination as shown in Table 2, and reacted. Subsequently, the resulting reaction mixture was added with EDAC (N-(3-dimethylanlinopropyl)-N'-ethylcarbodiimide hydrochloride, produced by Sigma Co., Ltd.) to give a final concentration of 0.1%, and reacted for additional two hours. After washing, the resulting mixture was floated in a final floating liquid (a 5 mM Tris, 0.04% (w/v) BSA (bovine serum albumin), and subjected to ultrasonic dispersion in an ultrasonic dispersion apparatus (produced by Olympus Corporation), thereby dispersing the latex particles.

### (3) Preparation of Latex Particle Labeling Antibody Pad

### The latex particle labeling antibody pad was prepared in the same manner as in Example 1.

### (4) Antibodies to be immobilized on Membrane

The same ones as prepared in Example 1 were employed.

### (5) Preparation of Immunochromatographic Assay Medium for Detection of Humane Cytokine

The medium was prepared in the same manner as in Example 1.

The antibodies were disposed on the medium in the same manner as in Example 1.

### (6) Detection of Humane Cytokine

The color of the immunochromatographic spots was visually observed, and at the same time the color was detected by means of a fluorescence detector. The results are shown in Table 2.

**Table 2**

| Number | Label-Attached Reagent (Antibody for Immobilization) | Latex Particle Color Tone | Visual Observation | Detection by means of Fluorescence Detector |
|---|---|---|---|---|
| 1 | Anti IL-1a Antibody | Red | The spot is small in area and appears blurring. | B |
| 2 | Anti IL-1b Antibody | Blue | The spot is small in area and appears blurring. | C |
| 3 | Anti IL-2 Antibody | Green | The spot is small in area and appears blurring. | C |
| 4 | Anti IL-4 Antibody | Pink | The spot is small in area and appears blurring. | B |
| 5 | Anti IL-6 Antibody | Yellow | The spot is small in area and appears blurring. | C |
| 6 | Anti IL-7 Antibody | Blue | The spot is small in area and appears blurring. | C |
| 7 | Anti IL-8 Antibody | Red | The spot is small in area and appears blurring. | C |
| 8 | Anti IL-10 Antibody | Pink | The spot is small in area and appears blurring. | B |
| 9 | Anti IL-12q70 Antibody | Yellow | The spot is small in area and appears blurring. | C |
| 10 | Anti GM-CFS Antibody | Green | The spot is small in area and appears blurring. | C |
| 11 | Anti IFNγ Antibody | Red | The spot is small in area and appears blurring. | B |
| 12 | Anti TNFα Antibody | Blue | The spot is small in area and appears blurring. | C |

The results show that detection by means of a fluorescence detector of Nos. 4 and 8 being arranged side by side, Nos. 2 and 6 being arranged side by side, and Nos. 7 and 11 being arranged side by side is ambiguous. It has proved that this comparative example, although colored latex particles are used and detection of the spot is carried out as in the invention, is unclear in the color and is unsuitable for detection of many kinds of substances.

### [Comparative Example 2]

Preparation of monoclonal antibodies, quantum dots, quantum dot labeling antibodies, quantum dot labeling antibody pads was carried out in the same manner as in Example 1.

Further, antibodies to be immobilized on the membrane were prepared also in the same manner as in Example 1.

### (1) Preparation of Immunochromatographic Assay Medium for Detection of Humane Cytokine

The immunochromatographic assay medium for detection of humane cytokine having the same structure as shown in Fig. 1 was prepared and employed.

The antibodies were disposed on the medium in the same manner as in Example 1.

As the membrane, a nitrocellulose membrane sheet (white) with a size of 3 cm x 10 cm (produced by Millipore Co., Ltd.) was employed. As shown in Fig. 3, the anti IL-1a antibody to be immobilized was coated in the form of line at a position distant from one end (this end was designated as an upstream end, and the other end as a downstream end) on the long side of the sheet, employing a positive pressure jetting device Biojet (produced by Bio Dot Co., Ltd.). Then, the anti IL-1b antibody to be immobilized, the anti IL-2 antibody to be immobilized, the anti IL-4 antibody to be immobilized, the anti IL-6 antibody to be immobilized, and a control antibody exhibiting reaction termination were coated at a position distant from that position in that order in the same way as the anti-IL-1 a antibody. Similarly, the anti IL-1a antibody to be immobilized, the anti IL-7 antibody to be immobilized, the anti IL-8 antibody to be immobilized, the anti IL-10 antibody to be immobilized, the anti IL-12q70 antibody to be immobilized, and the control antibody were coated on a second membrane, and the anti IL-1a antibody to be immobilized, the anti GM-CFS antibody to be immobilized, the anti IFNγ Antibody to be immobilized, and the anti TNFα antibody were coated on a third membrane. After that, the coatings were dried by spraying a 45°C hot air for ten minutes. Then, in order to fix the resulting materials and enhance the strength, a plastic backing sheet (produced by Bio Dot Co., Ltd.) was laminated on the surface (designated as under surface) of the membrane opposite the surface (designated as upper surface) with the coatings.

Subsequently, the quantum dot labeling antibody pad prepared in Example 1, item (4) was cut, and adhered on the upper surface of the membrane so that the pad overlapped with the membrane by 2 mm from the upstream end, thereby providing a sample dropping pad.

Subsequently, a cellulose filter paper with a size of 30 mm (width) x 10 cm (length) (produced by Watmann Co., Ltd.) was adhered on the upper surface of the membrane so that the filter paper overlapped with the membrane by 5 mm from the downstream end, thereby providing a sample absorption pad.

Subsequently, the entire upper surface was covered with a transparent plastic laminate (produced by Adhesive Research Co., Ltd.) except for an upstream end width of 7 mm of the sample dropping pad.

An immunochromatographic medium as shown in Fig. 3 was prepared, in which a first membrane was employed. Similarly, an immunochromatographic medium employing the second membrane and an immunochromatographic medium employing the third membrane were prepared.

### (2) Detection of Humane Cytokine

The detection was carried out in the same manner as in Example 1. Twelve kinds of antibodies were evaluated by visual observation and by means of a fluorescence detector. The results are shown in Table 3. Incidentally, evaluation by visual observation of the control antibodies (Nos. 6 and 11) were also shown in Table 3.

**Table 3**

| No. | Antibody | Membrane | Visual Observation | Detection by means of Fluorescence Detector |
|---|---|---|---|---|
| 1 | Anti IL-1a Antibody | 1 | Line is clearly observed. | A |
| 2 | Anti IL-1b Antibody | 1 | Line is clearly observed. | A |
| 3 | Anti IL-2 Antibody | 1 | Line is clearly observed. | A |
| 4 | Anti IL-4 Antibody | 1 | Line is clearly observed. | A |
| 5 | Anti IL-6 Antibody | 1 | Line is clearly observed. | A |
| 6 | Anti IL-1a Antibody | 2 | Line is clearly observed. | - |
| 7 | Anti IL-7 Antibody | 2 | Line is clearly observed. | A |
| 8 | Anti IL-8 Antibody | 2 | Line is clearly observed. | A |
| 9 | Anti IL-10 Antibody | 2 | Line is clearly observed. | A |
| 10 | Anti IL-12q70 Antibody | 2 | Line is clearly observed. | A |
| 11 | Anti IL-1a Antibody | 3 | Line is clearly observed. | - |
| 12 | Anti GM-CSF Antibody | 3 | Line is clearly observed. | A |
| 13 | Anti IFNγ Antibody | 3 | Line is clearly observed. | A |
| 14 | Anti TNFα Antibody | 3 | Line is clearly observed. | A |

As is apparent from Table 3, when the quantum dot label in the invention is used, every line is clearly observed, but the number of the kind of a substance to be detected is limited, and the upper limit of the number is five. Further, it has proved that in order to detect twelve kinds of the substance to be detected as in Example 1, three membranes are necessary in this comparative example, which is inferior in ease of detection and complicated to detect. 2) Detection of Humane Cytoline

As is apparent from comparison of Example 1 with comparative examples 1 and 2, the constitution of the invention has a great advantage that it can simultaneously detect many kinds of substances with clarity and with ease. It is expected that the excellent properties of the constitution of the invention make it possible to detect each spot quantitatively and bring about a great improvement in diagnosis according to immunochromatography.

### EXPLANATION OF SYMBOLS

a: Sample Dropping Pad
b: Membrane
c: Backing Sheet
d: Sample Absorption Pad
e: Laminate
f: Detection Portions In The Form Of Dots
g: Fluorescence Detector
h: Light Source
i: Control Antibody Coating Portion
j: IL-4 Antibody Coating Portion
k: IL-1b Antibody Coating Portion
l: IL-6 Antibody Coating Portion
m: IL-2 Antibody Coating Portion
n: IL-1a Antibody Coating Portion

## Claims

1. An immunochromatographic medium comprising a support, a label-attached reagent immobilized on a labeling agent, and detection portions on which a material capable of combining with a substance to be detected in a sample is immobilized, the label-attached reagent and the detection portions being provided on the support, featured in that the labeling agent is a phosphor nanoparticle and the detection portions are shaped in the form of dots.

2. The immunochromatographic medium of claim 1, featured in that the number of the dots of the detection portions is from 3 to 30.

3. An immunochromatographic method employing the immunochromatographic medium of claim 1 or 2, the method featured in that it comprises:
reacting a label-attached reagent, which is immobilized on a labeling agent and which is capable of combining with a substance to be detected in a sample, with a substance to be detected, thereby obtaining a chromatographic solution;
causing the chromatographic solution to migrate on the immunochromatographic medium to detection portions on which a material capable of combining with the substance to be detected is immobilized; and
capturing and detecting the substance to be detected at the detection portions.

4. The immunochromatographic method of claim 3, featured in that the labeling agent is a quantum dot.

5. The immunochromatographic method of claim 3 or 4, featured in that three to fifteen items of the substance to be detected can be detected simultaneously.
